# EUROPEAN PATENT SPECIFICATION

(11) **EP 2 875 809 B1**
(45) Date of publication and mention of the grant of the patent: **30.05.2018**
(21) Application number: 14194659.0
(22) Date of filing: 25.11.2014
(51) Int. Cl.: A61K 31/047, A61K 31/736, A61P 15/08, A61P 3/00, A61P 3/10, A61P 5/48

(54) **Product based on an association of glucomannan and inositol**
Produkt auf Basis von Vereinigung von Glucomannan und Inositol
Produit sur la base d'une association de glucomannane et d'inositol

(30) Priority: 26.11.2013 IT RM20130655
(43) Date of publication of application: 27.05.2015
(73) Proprietor: Dicofarm Spa, 00138 Roma (IT)
(72) Inventor: Acri, Maurizio, 00138 Roma (IT)
(74) Representative: Modiano, Micaela Nadia

(56) References cited:
- WO-A2-2007/019540
- CA-A1- 2 558 110
- VUKSAN V ET AL: "CHRONIC FEEDING OF KONJAC-MANNAN (KJM) FIBRE IMPROVES POSTPRANDIAL GLYCEMIA IN INSULIN RESISTANCE", FASEB JOURNAL, FED. OF AMERICAN SOC. FOR EXPERIMENTAL BIOLOGY, US, vol. 14, no. 4, 15 March 2000 (2000-03-15) , page A727, XP001057055, ISSN: 0892-6638
- GIORDANO D ET AL: "Effects of myo-inositol supplementation in postmenopausal women with metabolic syndrome: A perspective, randomized, placebo-controlled study", MENOPAUSE, LIPPINCOTT WILLIAMS & WILKINS, US, vol. 18, no. 1, 1 January 2011 (2011-01-01), pages 102-104, XP009153478, ISSN: 1072-3714, DOI: 10.1097/GME.0B013E3181E8E1B1
- NORDIO M ET AL: "The combined therapy with myo-inositol and D-chiro-inositol reduces the risk of metabolic disease in PCOS overweight patients compared to myo-inositol supplementation alone", RIVISTA EUROPEA PERLE SCIENZE MEDICHE E FARMACOLOGICHE // EUROPEAN REVIEW FOR MEDICAL AND PHARMACOLOGICAL SCIENCES // REVUE EUROPEENNE POUR LES SCIENCES MEDICALES ETPHARMACOLOGIQUES, ROME, IT, vol. 16, no. 5, 1 May 2012 (2012-05-01), pages 575-581, XP009168775, ISSN: 0392-291X
- DATABASE WPI Week 201313 Thomson Scientific, London, GB; AN 2012-R36390 XP002727129, -& CN 102 715 392 A (ZHANG W) 10 October 2012 (2012-10-10)

## Description

### Field of application

In its most general aspect, the present invention relates to nutraceutical products, dietary supplements and medical devices based on a combination of glucomannan and inositol (in the various isomeric forms thereof) to prevent or treat the main symptoms of polycystic ovary syndrome and metabolic syndrome.

### Background

Polycystic ovary syndrome (PCOS) is an endocrine and metabolic disease affecting 5-10% of female population worldwide, and therefore it represents the most frequent female endocrine disorder, in reproductive age, with gynecological consequences; it is also the most common cause of female infertility (1).

The syndrome diagnosis is performed on the basis of the presence of at least two of the following symptoms in the patient: (a) oligo-amenorrhea with oligo-anovulation, (b) clinical and/or biochemical hyperandrogenism, (c) presence of ovarian cysts in absence of other disorders causing ovarian cysts development (2, 3).

The symptoms expression for this syndrome is very variable. Some syndrome common signs and symptoms are: menstrual irregularities such as oligomenorrhea, with menstrual cycle lasting more than 35 days, and/or amenorrhea; infertility, in most cases related to chronic anovulation; androgenic alopecia; acne; oily skin and seborrheic dermatitis; acanthosis nigricans, i.e. presence of dark spots on the skin; presence of soft fibromas; prolonged periods of premenstrual syndrome, with symptoms such as bloating, mood swings, pelvic pain, back pain; high blood male hormones, i.e. androgens, and in particular testosterone, androstenedione and DHEAS, causing hirsutism and sometimes masculinization; polycystic ovaries possibly with increased ovaric volume; LH (luteinizing hormone) /FSH (follicle stimulating hormone) ratio greater than 2.5; low levels of SHBG (Sex hormone-binding globulin).

A recent study shows that women affected by polycystic ovary syndrome are more susceptible to other types of dysfunctions which however cannot be considered merely POCS symptoms; the most common and frequent are the metabolic syndrome symptoms, such as hyperinsulinemia and consequent impaired glucose tolerance, diabetes, cardiovascular problems, and visceral obesity, as well as the syndrome of obstructive sleep apnea.

The PCOS symptoms complexity and variability is due to complex hormonal alterations. PCOS patients have hyperandrogenism and hypoestrogenism The high androgen level is responsible for a failed/incomplete development of Graafian follicles, which, by cicatrizing, leave cysts.

The hormone levels alteration also causes other PCOS symptoms, including hirsutism and acne.

It has now been established that PCOS etiology is multifactorial and such multiple factors act causing the complex syndrome pattern described. However, it is still not clear what pathogenic event triggers the chain reaction. However, it seems to be assessed that, regardless which is the initial event, a condition of hyperandrogenism leads to an excessive production of estrone, which in turn determines a hyperproduction of gonadotropins.

So far, it has not been found any therapy able to treat completely and univocally the pathology; therapeutic choices are often focused on the resolution of associated symptoms shown by each patient. In fact, the presence of other symptoms due to the related diseases above mentioned, may influence the choice of the therapeutic approach to follow. Since most of the related dysfunctions have its own treatment, many specific treatments for such conditions are used in combination with the PCOS therapeutic means leading to break the vicious circle of hyperandrogenism and chronic anovulation.

Recent studies have shown that insulin resistance is an important etiopathogenic element for the syndrome and that hyperinsulinemia in PCOS patients significantly contributes to hyperandrogenism.

Frequently, the simple correction of the lifestyle by diet and physical activity, resulting in weight loss and reduction in fat mass, determines improvement in insulin resistance, a decreased peripheral production of androgens and a minor transformation of the latter into estrogen.

The assessment of the hyperinsulinemia critical role in the POCS has provided the rational for the introduction of new insulin-sensitizing drugs in the treatment of such patients.

Accordingly, besides the most commonly used therapeutic approaches for the treatment of PCOS, aimed to reduce the high androgen levels through the use of antiandrogens or oral contraceptives, nowadays therapies to increase insulin sensitivity are prescribed too. Such drugs improve the body's response to insulin secretion, reducing the amount of sugar circulating in the blood.

Consistent evidences have shown that a short-term use of these substances allows to:
- regulate period and ovulation,
- reduce infertility associated to polycystic ovary syndrome,
- reduce hirsutism,
- reduce acne.

Among the mediators of insulin used in the treatment of polycystic ovary syndrome there are the inositol-phosphoglicans.

Inositol is a molecule that could be found in nature in several chemical forms (isomers); the most common form is called myo-inositol.

Inositol is also found in many foods, in particular cereals, nuts, fruits, especially melons and oranges, as well as in meat. Inositol is involved in many biological functions, such as:
- regulation of sugar metabolism,
- stability of cell walls,
- regulation of certain functions related to the transmission of cellular signals, for example regulation of intracellular calcium concentrations and membrane potential,
- reduction of the cholestrolemia
- regulation of some gene expression mechanisms.

Recent findings has established the efficacy of inositol, and derivatives thereof, as a supplement in other areas, in the treatment of polycystic ovary. A therapy based on inositol has relatively low costs and practically no side effects compared to those caused by other drugs normally used such as, for example, metformin. Several studies have shown that one of the isomers of inositol, the D-chiro-inositol, thanks to its ability to increase insulin sensitivity, has beneficial effects on ovulation and androgens production in women with polycystic ovary syndrome. The administration of D-chiro-inositol has been associated with a reduction in serum testosterone concentration and an increasing of SHBG concentration. Simultaneously with the reduction of insulin secretion, women assuming D-chiro-inositol showed an improvement of ovulatory function. Supplementation with another inositol isomer, myo-inositol, helps to reduce the amount of FSH required for ovulation, improves oocyte quality, reducing germinal vesicle and degenerated oocyte, and increases the number of oocytes collected after ovarian stimulation in patients undergoing medically assisted procreation procedures. Several scientific evidences show that the combined use of the 2 isomers, in specific proportions, results in a greater efficacy than the single isomer use (4).

The inositol biological role also includes a specific activity controlling fats and sugars metabolism and cellular functionality of the nervous system; finally, it appears essential in the baldness prevention. Scientific studies have shown that diabetics eliminate an inositol amount significantly higher compared to non-diabetic subjects. In cases of insulin resistance or diabetes type II, inositol helps to improve the overall clinical feature; in this context inositol is useful to prevent and correct the pathophysiological mechanisms underlying the metabolic and reproductive anomalies related to polycystic ovary syndrome.

In a study designed to compare the efficacy of myo-inositol and metformin, alone or in combination with r-FSH in the treatment of menstrual irregularities, chronic anovulation and infertility in patients with polycystic ovary syndrome, either metformin and myo-inositol have been shown to represent the best treatment to restore normal period in patients with PCOS, however, from the obtained results the treatment based on myo-inositol seems to be more effective than the drug of the biguanides family, for the treatment of diabetes type 2 (6).

Glucomannan is extracted from the root of *Amorphophallus konjac,* a herbaceous perennial plant from Asia and Eastern Europe.

Currently, the interest of the Western countries towards the *Amorphophallus konjac* is addressed to its high content in glucomannan, a polysaccharide that in contact with water swells by absorbing up to 60 times its weight without cleaving into simple sugars it is composed by, glucose and mannose.

This phenomenon is favored by the stomach acid environment, wherein it forms a gelatinous mass with high viscosity, essentially calories free, especially useful as co-adjuvant in low-calories diets. Glucomannan, if ingested in large quantities of water, forms a viscous gel in the stomach producing a voluminous food bolus, smooth and soft, blended with the food introduced during the meal, which forms a non-digestible coating around the food particles, creating a barrier for nutrients trapped in the soft and viscose mass. Consequently, the absorption of food is hampered or reduced, since the gel partially prevents the contact of the food particles to the digestive enzymes action, in particular by determining a slowed absorption of carbohydrates and a reduction in lipids absorption, including cholesterol and triglycerides. The gelatinous mass, also, relaxes the gastric wall, producing a satiety effect. Subsequently, proceeding in the digestive tract, it constitutes a soft and hydrated mass in the intestinal lumen that is not absorbed, has no systemic effect, and by pressing on the enteric walls and exerting a kind of massage of the gut walls, stimulates peristalsis accelerating and facilitating intestinal transit, with a mild laxative action that helps to remove many toxins present in the lumen. Glucomannan exerts a sort of intestine purification and produces a prebiotic action for beneficial bacterial flora. In particular, the slowed down absorption of carbohydrates determined by glucomannan, avoids the post-prandial glucose rise in blood, which would cause a sudden demand for insulin by the body. Insulin is a hormone, secreted by pancreas, whose best-known function is to regulate the blood glucose levels.

A meal high in, refined sugar is quickly absorbed and is the strongest stimulus for insulin production, insulin immediately acts to bring down to physiological levels the glucose blood levels by stimulating the entry of glucose into cells, and indirectly causing an accumulation of lipids in adipocytes, increasing the volume of adipose tissue, with increase of the body weight.

It is evident, therefore, that the blood glucose control is important in fighting overweight and obesity, since the insulin released from pancreas causes, in addition to the increased production of fatty acids, an early recurrence of the sense of hunger, determined by the blood glucose levels reduction.

Glucomannan, slowing the carbohydrates absorption reduces the postprandial glucose peak, keeping blood glucose levels within physiological limits, and thus exerting a particularly useful action in case of hyperglycemia or type 2 diabetes.

The glucomannan also reduces cholesterol and fats absorption. In view of these properties and many randomized clinical trials that have proven it, glucomannan has been approved by EFSA (the European Authority for Food Safety) as an effective component in maintaining normal levels of cholesterol in the general population and as an adjuvant in energy-restricted diets for weight reduction (EFSA Journal 2009; 7 (9): 1258) .

VUKSAN ET AL: "CHRONIC FEEDING OF KONJAC-MANNAN (KJM) FIBRE IMPROVES POSTPRANDIAL GLYCEMIA IN INSULIN RESISTANCE",FASEB JOURNAL, FED. OF AMERICAN SOC. FOR EXPERIMENTAL BIOLOGY, US, vol. 14, no. 4, 15 March 2000 (2000-03-15), page A727, discloses the use of Konjac-mannan (glucomannan) to treat insulin resistance syndrome. The Canadian patent application CA2558110 describes a food supplement and a method to enhance or promote insulin activity, to stimulate skeletal muscle growth and at the same time reduce skeletal muscle loss comprising at least D-pinitol and leucine or its derivatives. According to the invention the dietary supplement can further comprise at least one of alpha lipoic acid, glucomannan, D- or myo-inositol, guar gum, taurine or their derivatives, a ketoisocaproic acid derivative, a derivative of alpha-ketoglutarate, and a source of C12 peptide.

The international patent application WO2005079857 concerns a method for increasing the bioavailability of nutrients using polysaccharides such as galactomannans and similar molecules to introduce active substances into human or animal metabolism, for example plant extracts, water-soluble and fat-soluble vitamins, amino acids, fatty acids, minerals, antioxidants and hormones, such as human growth hormone HGH and others. Claim 5 of the patent application specifies that the bioavailability of the following water-soluble vitamins is increased: vitamin B1, vitamin B2, niacin, vitamin B6, vitamin B12, folic acid, myo-inositol, pantothenic acid and vitamin C.

Moreover, in order to obtain the sustained release of a pharmaceutical substance, meanwhile maintaining high bioavailability and limiting any potential side effects, the Japanese patent application JPS63310827 involves the use of a polymeric substance water-soluble as a carrier for a sustained release. According to the invention the pharmaceutical composition for a prolonged release is achieved by adding a water-soluble, highly polymeric substance such as hydroxypropyl cellulose, carboxymethyl cellulose, methyl cellulose, pullulans, PVA and glucomannan, releasing a principal agent consisting of a derivative of nicotinic acid selected from nicotinamide, nicomol, epronicato, niceritrolo, myo-inositol hexanicotinate, etc..., if necessary, by mixing a lubricant, excipient, etc., and preparing the drug by a conventional method.

Although the effects of glucomannan and inositol in the sugar metabolism and blood sugar levels regulation are known, studies examining the effect of an inositol and glucomannan combination in relation to the treatment of the metabolic syndrome and the main symptoms of POCS have not been reported. The reasons to consider this effect and its possible application concern on having new, alternative and effective products for treating insulin resistance in women with POCS.

### Summary of the invention

The Applicant has found that the myo-inositol and glucomannan association, contributes to the improvement of the clinical symptoms of polycystic ovary syndrome. The combination of myo-inositol and glucomannan, which individually and in a different way exert an effect in the regulation of carbohydrate, synergistically determine an important effect on reducing the insulin levels in treated subjects, leading to a significantly lower level than those resulting from treatment with myo-inositol alone or glucomannan alone.

A first object of the invention is, therefore, an association of glucomannan, or its polymers and/or mixtures of polymers derived therefrom, and myo-inositol, and/or D-chiro-inositol for the use in the treatment of typical symptoms of the polycystic ovary syndrome.

A second object of the invention is a nutraceutical preparation, and/or food supplement, and/or medical compounds for the use in the treatment of typical symptoms of polycystic ovarian syndrome comprising the association of glucomannan, and/or its polymers and/or mixtures of polymers therefrom derived, and myo-inositol, and/or D-chiro-inositol.

### Figure description

- Figure 1 shows the graph of the insulin amount detected in treated patients of the three groups of study;
- Figure 2 shows the graph of the glucose detected in treated patients of the three groups of study.

### Detailed description of the invention

The present invention provides compositions, nutraceutical and medical compounds, dietary supplements suitable to prevent and treat the typical main symptoms of polycystic ovary syndrome and the metabolic syndrome.

In particular, the invention contributes to the improvement and regression of the main symptoms related by lowering the blood glucose level and basal insulin that is known to be one of the etiological factors of POCS. The composition and products according to the invention are characterized by comprising, as active ingredient, a combination of glucomannan and inositol as defined in the claims. The association can comprise all inositol isomers and/or mixtures of inositol isomers, and all the polymers of glucomannan and/or mixtures of polymers derived thereof, as defined in the claims. According to the present invention the combination comprises an amount of glucomannan sufficient to delay the pharmacokinetics of inositol, in fact glucomannan, having the property to delay the sugar absorption, is able to prolong the inositol action, and/or its isomers present in the association. The synergistic action of the two components determines an enhanced effect on sugar metabolism regulation compared to the one exerted by the two components individually.

The glucomannan of the present invention can be derived from a natural source or from a synthetic process, but preferably it is derived from natural source, in particular from *Amorphophallus konjac* root, a perennial herbaceous plant, containing high amount of glucomannan.

The composition according to the invention is suitable for oral administration and is prepared in solid form, powder, granules, capsules, tablets, etc..

The composition according to the invention may further comprise pharmaceutical acceptable excipients such as for example, and as a non-limiting example, dispersing agents, agents, carriers, flavoring and coloring.

In a particularly preferred embodiment of the invention the composition comprises glucomannan powder in an amount ranging from 0 to 20 g, myo-inositol powder in an amount ranging from 0 to 10 g and D-chiro-inositol in an amount ranging from 0 mg to 5 g and is in a form of capsule or powder.

In a particularly preferred embodiment the composition comprises 1 g of myo-inositol, 125 mg of D-chiro-inositol and 2 g of glucomannan, representing the dose suitable for an oral administration.

In a further embodiment the composition according to the invention comprising the association of glucomannan powder, myo-inositol powder and D-chiro-inositol further comprise manganese.

In a preferred embodiment the composition comprises 1-5 g of glucomannan, 100-300 mg of D-chiro-inositol and 1-5 g of myo-inositol and 1-50 mg of manganese.

In a particularly preferred embodiment the composition comprises 1 g of myo-inositol, 125 mg of D-chiro-inositol, 2 g of glucomannan and 10 mg of manganese, representing the dose suitable for an oral administration.

Further characteristics and advantages of the present invention will appear clearly from the following examples reported for illustrative purpose.

### EXPERIMENTAL PART

The following examples illustrate the experimental use *in vitro* of the invention.

### Preparation of the composition characterized by the association of myo-inositol and glucomannan

A composition was prepared comprising:
glucomannan powder 0-20 g
myo-inositol powder 0-10 g
Effectiveness Evaluation

In order to evaluate the effectiveness of this new combination of inositol and glucomannan on blood glucose levels and insulin at baseline and after glucose load curve, it was evaluated the effect in 20 women affected by insulin resistance PCOS and compared with what determined in 2 groups of 10 women each, treated with myo-inositol and glucomannan individually administered.

### Materials and methods

Forty women, aged between 20 and 30 years affected by polycystic ovary syndrome (PCOS) who had an anovulatory cycle with menstrual irregularities, acne and hirsutism with a body mass index > 25, were enrolled in the study. The women were divided into 3 groups, each group received the treatment according to the following scheme:
Group A: 20 women treated with 1.5 g of myo-inositol + 2 g of glucomannan, twicea day for 3 months, administered half an hour before meals;
Group B: 10 women treated with 1.5 g myo-inositol, twice a day for 3 months, administered half an hour before meals;
Group C: 10 women treated with a dose of 2 g of glucomannan, twice a day for 3 months, adminisered with main meals.

In order to determine blood glucose and insulin levels in all samples, OGTT (oral glucose tolerance test) was performed in all women in the early follicular phase of the spontaneous period, or after a progestin-induced, period before starting the treatment and after three months of treatment (OGTT 75 g at 0, 30, 60, 90 and 120 min) .

In addition to the assessment of blood glucose and insulin levels during OGTT, the menstrual cycle trend has been evaluated in all the women: the 3^{rd} month of treatment at the 22^{nd} day of the menstrual cycle a progesterone dosage was performed and, after 3 months of treatment, an evaluation of acne, hirsutism and body mass index was performed.

### RESULTS

All patients completed the study protocol and no side-effects were referred except for a slight nausea in women belonging to the group that assumed the association myo-inositol-glucomannan.

In Table 1 are shown the values of the insulin levels in the three groups of patients.

**Table 1**

| Time | Group A | Group B | | Group C | |
|---|---|---|---|---|---|
| | myo-inositol-glucomannan | myo-inositol | P | glucomannan | P |
| 0 | 7.3±1.2 | 7.6+1. | 0.7 | 10.5±2.2 | 0.090 |
| 30' | 47+8,13 | 64.1+5,66 | 0.0187 | 64±10.51 | 0.0243 |
| 60' | 42.35±12.03 | 57.5±7.13 | 0.041 | 54.9±8.67 | 0.0282 |
| 90' | 38.45±8.23 | 48.4±4.81 | 0.0922 | 46.2±5.71 | 0.1727 |
| 120' | 26±7.36 | 40.7±5.57 | 0.1027 | 41±4.47 | 0.0043 |
| 180' | 23.4±4.77 | 34±4.89 | 0.0124 | 29.5±7.9 | 0.3008 |

In Table 2 are shown the values of blood glucose levels in the three groups of patients.

**Table 2**

| Time | Group A | Group B | | Group C | |
|---|---|---|---|---|---|
| | myo-inositol-glucomannan | myo-inositol | P | glucomannan | P |
| 0 | 84.9±6.67 | 90±4.71 | 0.4139 | 88.8±7.84 | 0.9593 |
| 30' | 113±5.95 | 130.8±6.56 | 0,0058 | 134.7±9.36 | 0.0059 |
| 60' | 107.5±5.96 | 122.6:1:11.7 | 0.0246 | 137.5±7.69 | 0.0020 |
| 90' | 96±4.18 | 107.2±10.25 | 0.0282 | 115.4±4.42 | 0.0059 |
| 120' | 86.2±4.79 | 98±6.92 | 0.0059 | 101.6±4.42 | 0.0020 |
| 180' | 77.15±4.3 | 91.1±6.15 | 0.0080 | 91.9±2.28 | 0.0053 |

The results showed a significant reduction in blood glucose levels and insulin levels after OGTT in all three groups, but the reduction was significantly greater in group A treated with the combination of myo-inositol and glucomannan. In particular, the association of myo-inositol anf glucomannan resulted to be effective in reducing the basal glicemic levels in a fasting state and during the ogtt in a linear trend as well as limiting insulin increasing. This specific trend is due to the synergistic action of the two substances that, by their mechanism of action, from one hand has fostered a reduced availability of glucose and from the other hand has improved insulin sensitivity, thus it was significantly reduced the insulin releasing from pancreas.

Group B, who has received only glucomannan, reported a good result in terms of reduction of both blood glucose levels and insulin, demonstrating that the use of this fiber may play a role in the treatment of insulin-resistant women with PCOS.

The women of group C, who have used only myo-inositol, have reported a reduction in blood glucose and insulin levels lower than the association myo-inositol-glucomannan.

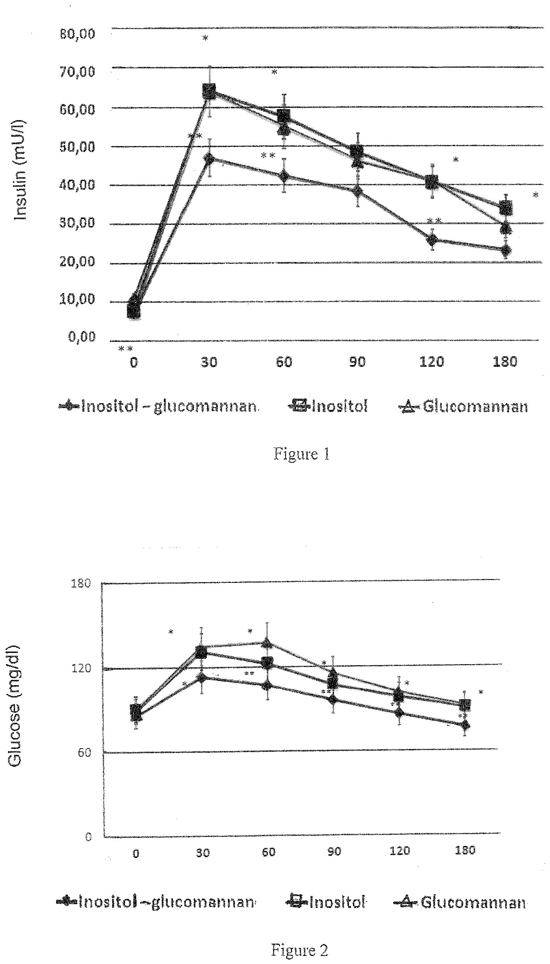

The results obtained are graphically represented in Figures 1 and 2.

Regarding the effectiveness of the association on the menstrual cycle, all patients reported an improvement of the menstrual cyclicity during the 3 different treatments. The frequency of the period was between 31 and 34 days in group B compared to 38-42 days, and between 32 and 35 days in group C compared to 39-44 days. The best result was observed in women of group A treated with the combination of myo-inositol-glucomannan wherein a normalization of the period, from 26 to 30 days, was found in comparison to the situation before the treatment, when it was from 36 to 45 days. The difference was found to be more significant in group A compared to groups B and C (p <0.01).

The evaluation of the menstrual cycle normalization in the treated patients was also performed by determining plasma progesterone at the 22^{nd} day of the period during the 3^{rd} month of treatment. In group A, a significantly greater increased level (p <0.01) compared to groups B and C (group A: 2.0 ± 0.8 ng/ml to 11.5 ± 1.2 ng/ml, group B: 2.2 ± 0.2 ng/ml to 4.2 ± 0.6 ng/ml, group C: 3.7 ± 0.9 ng/ml to 4.6 ± 1.2 ng/ml) was detected. These data demonstrated the complete normalization of the ovulatory activity in women belonging to group A compared to groups B and C.

An adequate ovulatory phase is defined when progesterone levels, measured in the medium luteal phase, are greater than 10 ng/ml.

The body mass index was significantly reduced in group A after treatment with the combination of the invention (inositol + glucomannan) with values from 28 + 2 to 23 ± 1.2 at the 3^{rd} month of treatment. The assessment of body mass index in group B showed a lower reduction, from 27 ± 0.6 to 25 ± 1.3 at month 3, as well as in group C wherein values decreased from 28 ± 1.3 to 26 ± 0.7 at the 3rd month of treatment.

All the considered parameters evaluated in the three groups resulted to be significantly improved in the group A, and in particular with regard to the body mass index with the recovery of the normal range (<24.9) in all women treated with the association myo-inositol-glucomannan.

The assessment of acne and hirsutism showed no significant changes in all 3 groups of treated women; this is explained by the short period of observation, as the parameters related to hyperandrogenism need a longer treatment time, since the first improvements are usually observed after 120-150 days even with the standard antiandrogen therapies.

The data obtained by the use of the association showed a better results than expected and encourage the use in the treatment of women with PCOS insulin resistant.

The novel character of the invention is consisting in the association of an isomer of inositol with glucomannan, which, through a synergistic action, is able to control insulin resistance. The fiber of glucomannan, in addition to delay the absorption of sugars in the intestine, is able to slow the absorption of the myo-inositol prolonging its action. This action can improve the inositol pharmacokinetics letting it acquire a prolonged activity resulting to induce a more rapid weight loss in overweight PCOS women.

Therefore, in light of the present results this association represents a new and more effective treatment for women with PCOS and insulin resistance.

Moreover, the absence of side effects confirms the safety of the two substances and assures physicians and women about their use.

### LITERATURE

1. De Leo V, La Marca A, Petraglia F. 2003, Insulin-Lowering Agents in the Management of Polycystic Ovary Syndrome; by The Endocrine Society.
2. The Rotterdam ESHRE/ASRM-sponsored PCOS consensus workshop group. (2004) Revised 2003 consensus on diagnostic criteria and long-term health risks related to polycystic ovary syndrome (PCOS). Hum Reprod 19, 41-47.
3. Franks S, McCarthy MI, Hardy K. Development of polycystic ovary syndrome: involvement of genetic and environmental factors. 2006. Int J Androl.; 29:278-85.
4. Nordio M, Proietti E. 2012. The combined therapy with myo-inositol and D-chiro-inositol reduces the risk of metabolic disease in PCOS overweight patients compared to myo-inositol supplementation alone. Eur Rev Med Pharmacol Sci. 16:575-81.
5. Chearskul S. et al. Glycemic and lipid responses to glucomannan in Thais with type 2 diabetes mellitus. J. Med. Assoc. Thai. 2007; 90: 2150-7.
6. Raffone E., Rizzo P., Benedetto V. 2010. Insulin sensitiser agents alone and in co-treatment with r-FSH for ovulation induction in PCOS women. Gynecological Endocrinology, 26, 275-280.

## Claims

1. Composition for the use in the treatment and prevention of the main symptoms of polycystic ovary syndrome and metabolic syndrome, **characterized by** comprising as active ingredient the association of glucomannan and myo-inositol or D-chiro-inositol or a mixture thereof.

2. Composition for use according to claim 1, **characterized in that** it comprises the association of myo-inositol or D-chiro-inositol or a mixture thereof with polymers of glucomannan and/or mixtures of polymers derived from glucomannan.

3. Composition for use according to claim 1 and 2 wherein the amount of glucomannan is sufficient to delay the pharmacokinetics of the inositol isomers.

4. Composition for use according to claims 1 to 3 **characterized in that** it comprises an amount of glucomannan lying in the range from 0.05 g to 20 g, an amount of myo-inositol lying in the range from 0.05 g to 10 g, an amount of D-chiro-inositol lying in the range from 0.05 mg to 5 g.

5. Composition for use according to claims 3 and 4 **characterized in that** it preferably comprises 2g of glucomannan, 1.0 g of myo-inositol and 125 mg of D-chiro-inositol.

6. Composition for use according to claims 1 to 5 **characterized in that** it further comprises manganese.

7. Composition for use according to claims 1 to 5 **characterized in that** the glucomannan is derived from a natural source, preferably from *Amorphophallus konjac* root.

8. Composition for use according to claims from 1 to 5 **characterized in that** the glucomannan is obtained by a synthetic process.

9. Composition for use according to any of the previous claims **characterized in that** it further comprises acceptable pharmaceutical excipients, such as dispersants, carrier, flavorings and colorings.

10. Composition for use according to any of the previous claims **characterized in that** it is suitable for oral administration.

11. Composition for use according to claim 10 **characterized in that** it is in solid form, powder, granules, capsules, tablets, in sachets for extemporaneous administration.

## Patentansprüche

1. Zusammensetzung zur Verwendung in der Behandlung und Vorbeugung der Hauptsymptome von polyzystischem Ovarsyndrom und Stoffwechselsyndrom, **dadurch gekennzeichnet, dass** sie als Wirkstoff die Assoziation von Glucomannan und Myo-Inositol oder D-Chiro-Inositol oder eine Mischung davon umfasst.

2. Zusammensetzung zur Verwendung gemäß Anspruch 1, **dadurch gekennzeichnet, dass** sie die Assoziation von Myo-Inositol oder D-Chiro-Inositol oder eine Mischung davon mit Polymeren von Glucomannan und/oder Mischungen von Polymeren umfasst, die von Glucomannan abgeleitet sind.

3. Zusammensetzung zur Verwendung gemäß den Ansprüchen 1 und 2, worin die Menge an Glucomannan ausreicht, um die Pharmakokinetik der Inositol-Isomere zu verzögern.

4. Zusammensetzung zur Verwendung gemäß den Ansprüchen 1 bis 3, **dadurch gekennzeichnet, dass** sie eine Menge an Glucomannan umfasst, die im Bereich von 0,05 g bis 20 g liegt, eine Menge an Myo-Inositol, die im Bereich von 0,05 g bis 10 g liegt, eine Menge an D-Chiro-Inositol, die im Bereich von 0,05 mg bis 5 g liegt.

5. Zusammensetzung zur Verwendung gemäß den Ansprüchen 3 und 4, **dadurch gekennzeichnet, dass** sie vorzugsweise 2 g Glucomannan, 1,0 g Myo-Inositol und 125 mg D-Chiro-Inositol umfasst.

6. Zusammensetzung zur Verwendung gemäß den Ansprüchen 1 bis 5, **dadurch gekennzeichnet, dass** sie weiter Mangan umfasst.

7. Zusammensetzung zur Verwendung gemäß den Ansprüchen 1 bis 5, **dadurch gekennzeichnet, dass** das Glucomannan abgeleitet ist von einer natürlichen Quelle, vorzugsweise von *Amorphophallus konjac-Wurzel.*

8. Zusammensetzung zur Verwendung gemäß den Ansprüchen 1 bis 5, **dadurch gekennzeichnet, dass** das Glucomannan durch ein synthetisches Verfahren hergestellt wird.

9. Zusammensetzung zur Verwendung gemäß einem beliebigen der obigen Ansprüche, **dadurch gekennzeichnet, dass** sie weiter verträgliche pharmazeutische Bindemittel, wie zum Beispiel Dispergiermittel, Träger, Geschmacks- und Farbstoffe umfasst.

10. Zusammensetzung zur Verwendung gemäß einem beliebigen der obigen Ansprüche, **dadurch gekennzeichnet, dass** sie für die orale Verabreichung geeignet ist.

11. Zusammensetzung zur Verwendung gemäß Anspruch 10, **dadurch gekennzeichnet, dass** sie in fester Form, Pulver, Körnchen, Kapseln, Tabletten, in Beuteln zur Sofort-Verabreichung vorliegt.

## Revendications

1. Composition pour l'utilisation dans le traitement et la prévention des symptômes principaux de polykystose ovarienne et de syndrome métabolique, **caractérisée en ce qu'**elle comprend, comme ingrédient actif, une association de glucomannane et de *myo*-inositol ou de D-*chiro*-inositol ou d'un mélange de ces composés.

2. Composition pour utilisation conforme à la revendication 1, **caractérisée en ce qu'**elle comprend une association de *myo*-inositol ou de D-*chiro*-inositol ou d'un mélange de ces composés, avec des polymères de glucomannane et/ou des mélanges de polymères issus du glucomannane.

3. Composition pour utilisation conforme à la revendication 1 et la revendication 2, dans laquelle le glucomannane se trouve en une quantité suffisante pour retarder la pharmacocinétique des isomères d'inositol.

4. Composition pour utilisation conforme aux revendications 1 à 3, **caractérisée en ce qu'**elle renferme une quantité de glucomannane située dans l'intervalle allant de 0,05 g à 20 g, une quantité de *myo*-inositol située dans l'intervalle allant de 0,05 g à 10 g, et une quantité de D-*chiro*-inositol située dans l'intervalle allant de 0,05 mg to 5 g.

5. Composition pour utilisation conforme à la revendication 3 et la revendication 4, **caractérisée en ce qu'**elle contient, de préférence, 2 g de glucomannane, 1,0 g de *myo*-inositol et 125 mg de D-*chiro*-inositol.

6. Composition pour utilisation conforme aux revendications 1 à 5, **caractérisée en ce qu'**elle comprend en outre du manganèse.

7. Composition pour utilisation conforme aux revendications 1 à 5, **caractérisée en ce que** le glucomannane est d'origine naturelle et provient, de préférence, de racine d'*Amorphophallus konjac.*

8. Composition pour utilisation conforme aux revendications 1 à 5, **caractérisée en ce que** le glucomannane est obtenu au moyen d'un procédé de synthèse.

9. Composition pour utilisation conforme à l'une quelconque des revendications précédentes, **caractérisée en ce qu'**elle comprend en outre des excipients pharmaceutiques admissibles, tels que des agents dispersants, véhicules, agents aromatisants et colorants.

10. Composition pour utilisation conforme à l'une quelconque des revendications précédentes, **caractérisée en ce qu'**elle convient pour l'administration par voie orale.

11. Composition pour utilisation conforme à la revendication 10, **caractérisée en ce qu'**elle se présente sous forme solide, en poudre, granulés, gélules ou comprimés, ou en sachets pour administration extemporanée.
